(19) Europäisches Patentamt
European Patent Office
Office européen des brevets

(11) Publication number: **0 169 666 B1**

# (12) EUROPEAN PATENT SPECIFICATION

(45) Date of publication of the patent specification: **27.01.88**

(51) Int. Cl.⁴: **C 07 C 43/13**, C 07 C 41/01, B 01 J 31/20

(21) Application number: **85304436.0**

(22) Date of filing: **20.06.85**

## (54) Catalysts and process for the dealkoxyhydroxymethylation of acetals to form glycol ethers.

(30) Priority: **21.06.84 US 623059**
**21.06.84 US 622817**

(43) Date of publication of application:
**29.01.86 Bulletin 86/5**

(45) Publication of the grant of the patent:
**27.01.88 Bulletin 88/4**

(84) Designated Contracting States:
**BE DE FR GB IT NL**

(56) References cited:
**EP - A - 0 013 464**
**DE - A - 2 655 406**
**US - A - 4 390 734**

(73) Proprietor: **Sun Refining and Marketing Company, 1801 Market Street, Philadelphia, PA 19103 (US)**

(72) Inventor: **Duggan, D. Michael, 311 South Darlington Street, West Chester, PA. 19380 (US)**
Inventor: **Lyons, James E., 211 Cooper Drive, Wallingford, PA. 19086 (US)**
Inventor: **Myers, Harry K.,Jr., Limestone Road, Cochranville, PA. 19330 (US)**

(74) Representative: **Lewin, John Harvey et al, Eikington and Fife High Holborn House 52/54 High Holborn, London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 169 666 B1

**Description**

The invention as defined in the claims, relates to the dealkoxyhydroxymethylation of acetals. (The term «dealkoxyhydroxymethylation» herein includes dearyloxyhydroxymethylation). More particularly, it relates to a novel process for the dealkoxyhydroxymethylation of certain dialkyl-, dicycloalkyl-, or diaryl-aldehyde acetals by reacting said acetals with syngas, i.e., hydrogen and carbon monoxide, in the presence of either a carbyne-substituted cobalt carbonyl metal cluster catalyst alone, or promoted with a ruthenium carbonyl compound, phosphite-ruthenium promoted cobalt carbonyl catalyst systems, to form the corresponding glycol monoethers. Still more particularly, it relates to the catalysts per se. In a further embodiment, it is also directed to a process for the dealkoxyhydroxymethylation of acetals in the presence of a solvent in combination with the aforedescribed catalyst system. The acetals described herein may be added directly or formed in situ from the corresponding aldehyde and alcohol precursors.

The glycol ethers described herein encompass known classes of compounds having various uses, as for example as jet fuel additives, cleaners, coatings solvents, and intermediates in the production of certain diphthalates.

One current well-known method of manufacturing ethylene glycol monoethers such as monoalkyl ethers consists of reacting ethylene oxide with the alcohol corresponding to the desired alkyl ether, employing various known catalyst systems.

Alternatively, the cobalt-catalyzed reaction of aldehydes or their dialkyl acetals with syngas, i.e., a carbon monoxide-hydrogen mixture, to form the corresponding glycol ether is also described in the art. Thus, for example, a method of making ethylene glycol ethers is described in U.S. Patent No. 2,525,793 which employs cobalt oxide to catalyze the reaction of methylal with syngas to provide a reaction mixture which, after hydrogenation over nickel, gives relatively uneconomical conversions on the order of 25-33%.

Numerous attempts have been made to obtain more practical yields of glycol ethers from aldehydes or their dialkylacetals. A number of promoters have been used in conjunction with various cobalt catalysts in an effort to improve reaction rates and product yields. U.S. Patent 4,062,898, for example, discloses a ruthenium chloride-promoted cobalt iodide catalyst which hydrocarbonylates formaldehyde dimethylacetal (methylal) to ethylene glycol monomethyl ether (EGMME) in yields of 10% or less. The reaction temperature required is 185°C at 20 atm. or above. A second method, described in Jpn. Kokai Tokkyo Koho 81 83, 432 (1981) uses substantial quantities of 2,4,6- collidine or similar aromatic amines to promote the cobalt carbonyl-catalyzed hydrocarbonylation of methylal in benzene as a solvent. The reaction of methylal with highly pressurized syngas in this process at 190°C for 10 hours gave 44% selectivity to EGMME at 98% conversion. A further patent, Euro. Pat. Appln. EP 34,374 (1981) uses both iodine and triphenyl or tricyclohexylphosphine together with $RuCl_3 \cdot H_2O$ to promote the $Co(Ac)_2 \cdot 4H_2O$- catalyzed hydrocarbonylation of methylal using 3000 psig of syngas, and temperatures of between 150 and 175°C to obtain results nearly comparable to those of the Japanese.

More recently, Knifton has found that cobalt carbonyl promoted with a Group VIB donor ligand catalyzes the hydrocarbonylation of an aldehyde in an alcohol to make ethylene glycol monoethers; U.S. 4,308,403. Yields of ethylene glycol monobutyl ether (EGMBE) as high as 61% were reported in this patent. A cyclopentadienyl-ligated cobalt catalyst is also effective for these reactions giving glycol ethers in up to 54% yield; U.S. 4,317,943.

Propylene glycol monoalkyl ethers are formed by contacting high pressure mixtures of carbon monoxide and hydrogen with either an acetal or an aldehyde and an alcohol using a cobalt catalyst promoted with a tin- or germanium-containing compound; U.S. 4,356,327. Yields of glycol ethers up to 31% were reported in this patent. Ethylene glycol ethers were also formed from a formaldehyde acetal or formaldehyde and an alcohol using tin or germanium promoters for cobalt carbonyl; U.S. 4,357,477. The highest glycol ether yield (EGMBE) was 53% in this case.

Finally, propylene glycol monoalkyl ethers were formed by hydrocarbonylation of acetaldehyde acetals or acetaldehyde and alcohols using rhodium, ruthenium or nickel compounds to promote ether cobalt carbonyls or cobalt compounds having group V ligand systems attached.

Glycol ether yields up to 28% were realized when these promoters were used; Knifton, U.S. 4,390,734 (1983).

Thus, the use of various promoters for the cobalt-catalyzed hydrocarbonylation of aldehydes or acetals has resulted in glycol ether yields of from 10-61%, depending on the glycol ether produced. The highest reported yield of EGMME is 44%, of EGMBE is 61% and propylene glycol monoethyl ether, PGMEE is 28%.

In accordance with the present invention, there is provided an improved process for the reaction of certain dialkyl-, dicycloalkyl-, or diarylaldehyde acetals or their aldehyde and alcohol precursors with syngas in the presence of carbyne-substituted cobalt carbonyl metal cluster catalyst, $R^3CCo_3(CO)_9$ alone or promoted with $Ru_3(CO)_{12}$, wherein $R^3$ is as defined in the claims. Alternatively, these and other cobalt-ruthenium catalysts may be used in combination with certain defined organophosphites, as defined in the claims, to form the corresponding ethylene glycol monoethers. This reaction, which may best be described as the dealkoxyhydroxymethylation of an acetal formed separately or in situ by the known reaction of an aldehyde with an alcohol, may be depicted by the following general reaction scheme.

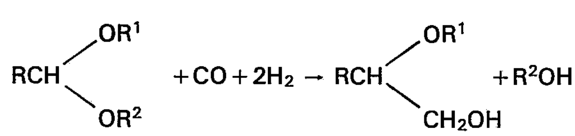

wherein R, $R^1$ and $R^2$, which may be the same or different comprise any organic moieties which are inert to the conditions of the reaction, and are selected from the groups consisting of:

a) a straight or branched chain alkyl group having from 1 to about 20 carbon atoms, such as methyl, ethyl, propyl, isopropyl, n-butyl, t-butyl, 2-ethylhexyl, dodecyl, and the like;

b) a substituted or unsubstituted cycloalkyl group having from 5 to about 20 carbon atoms, such as cyclopentyl, cyclohexyl, cycloheptyl, 3-methylcyclopentyl, 3-butyl cyclohexyl, cyclooctyl, adamantyl, decalyl, 3-phenylcycloheptyl and the like; and

c) a substituted or unsubstituted aryl group having from 6 to about 20 carbon atoms such as benzyl, phenyl, naphthyl, fluoroanthryl, tetralyl, tolyl, ethylphenyl, cumyl, anisyl, chlorophenyl, and the like;

and R may additionally be hydrogen.

It will be understood that when $R^1$ and $R^2$ in the foregoing reaction scheme are different, the resulting products will actually be mixtures of the corresponding ethers and alcohols. Also, as described in detail below, $R^1$ and $R^2$ may be joined by one or more bridging atoms to form a cyclic compound.

In a further embodiment of this invention, it has been found that when the aforedescribed dealkoxyhydroxymethylation process is carried out in the presence of a solvent in combination with the catalysts of this invention, significant additional improvements over prior processes are achieved.

The present process, using the novel catalysts of this invention, provides an improvement over the methods of the prior art in that these catalysts permit the reaction to be carried out under more mild conditions of time and temperature than those of the prior art, yet most surprisingly provide rates and selectivities of desired product over those obtained by the use of cobalt carbonyl alone, or the ruthenium carbonyls alone.

This invention is also directed to certain novel catalyst compositions comprising

(a) $R^3CCo_3(CO)_9$ promoted with $Ru_3(CO)_{12}$; or consisting essentially of

(b) a mixture of:

(I) an organophosphite promoter of the formula

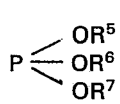

wherein $R^5$, $R^6$, and $R^7$, which may be the same or different, comprise any organic moieties which are inert to the conditions of the reaction, and are selected from the group consisting of:

1) hydrogen;

2) a straight or branched chain alkyl group having from 1 to about 20 carbon atoms, such as methyl, ethyl, propyl, isopropyl, n-butyl, t-butyl, 2-ethylhexyl, dodecyl, and the like;

3) a substituted or unsubstituted cycloalkyl group having from 5 to about 20 carbon atoms, such as cyclopentyl, cyclohexyl, cycloheptyl, 3-methylcyclopentyl, 3-butylcyclohexyl, cyclooctyl, adamantyl, decalyl, 3-phenylcycloheptyl and the like; and

4) a substituted or unsubstituted aryl group having from 6 to about 20 carbon atoms such as benzyl, phenyl, naphthyl, fluoroanthryl, tetralyl, tolyl, ethylphenyl, cumyl, anisyl, chlorophenyl, and the like; and

(II) a ruthenium promoted cobalt carbonyl compound or mixture of ruthenium carbonyl and cobalt carbonyl compounds selected from the group consisting of

1) a mixture of $Co_2(CO)_8$ and $Ru_3(CO)_{12}$;

2) $Co_2Ru(CO)_{11}$; and

3) a mixture of $R^3CCo_3(CO)_9$ promoted with $Ru_3(CO)_{12}$,

wherein $R^3$ may be hydrogen; $C_{1-12}$ alkyl; preferably $C_{1-5}$ lower alkyl; $C_{5-10}$ cycloalkyl or alkyl-substituted cycloalkyl; $C_{6-12}$ cycloalkenyl, such as cyclohexenyl or cyclooctenyl; $C_{1-12}$ alkoxy, such as methoxy or propoxy; $C_{6-20}$ aryl or alkyl-, cycloalkyl-, alkoxy-, halo-, or cyano-substituted aryl; or a silyl carbyne moiety of the formula $R_3^4Si$, wherein $R^4$ is alkyl or aryl.

The novel homogeneous catalyst systems employed in the process of this invention, i.e., the combination of the cobalt-ruthenium compounds and the organophosphites, are readily prepared by simply mixing the organophosphite promoter with any of the ruthenium-promoted cobalt carbonyl compounds or mixtures set forth above. The mole ratio of organophosphite to ruthenium-cobalt carbonyl compound or mixture is desirably in the range of 1:5 to 5:1, and preferably 1:3 to 3:1.

The organophosphite promoters are generally known compounds which are articles of commerce available to those skilled in the art. Examples of such phosphites include trimethylphosphite, triethylphosphite, tributylphosphite, triphenylphosphite, trineopentylphosphite, and tricyclohexylphosphite, of which the heavier molecular weight trialkylphosphites are preferred.

The first of the ruthenium promoted cobalt carbonyl catalysts which may advantageously be promoted by an organophosphite are ruthenium carbonyl-promoted dicobalt octacarbonyls, more specifically, triruthenium dodecacarbonyl-dicobalt octacarbonyl mixtures. This catalyst system is readily prepared by simply mixing dicobalt octacarbonyl ($Co_2(CO)_8$) with ruthenium dodecacarbonyl ($Ru_3(CO)_{12}$) in the reaction medium. The molar ratios of these two components, are optimally in the range of about 10:1 to 1:10, and preferably about 5:1 to 1:5.

The second of these catalysts consists of the metal cluster $Co_2Ru(CO)_{11}$. This catalyst may be prepared by the method disclosed by Roland et al., Angew. Chem. Int. Ed. Engl., 20, 679 (1981).

The third of these catalysts is a carbyne-substituted metal cluster having the formula $R^3CCo_3(CO)_9$, wherein $R^3$ is as defined above. These cobalt carbonyl compounds may be prepared in accordance with the procedures taught in Inorganic Synthesis, Wiley-Interscience Pub., New York, Vol. 20, #53-B, pp. 226 et seq. (1980). As

indicated above, this catalyst may be used alone, or, more desirably, in combination with $Ru_3(CO)_{12}$, a known compound. When used in combination, the molar ratios of these two carbonyl components, should optimally be in the range of about 10:1 to 1:10 and preferably about 5:1 to 1:5.

The acetal dealkoxyhydroxymethylation reaction with syngas, as described above, utilizing the ruthenium promoted cobalt carbonyl catalyst of this invention, with or without the phosphites, may conveniently be conducted in a generally known manner whereby the desired acetal is reacted with syngas under elevated temperature and pressures for given periods of time, during which period the reaction mixture is efficiently stirred. In this reaction, the volume ratio of carbon monoxide to hydrogen in the syngas desirably is in the range of from about 1:3 to 3:1, and more preferably 1:2 to 2:1. Following rapid cooling, the reaction product is then recovered from the mixture in a routine manner.

In contrast to the prior art reaction conditions described above, the catalysts of this invention advantageously permit the use of more mild operating conditions. Thus, temperatures in the range of from about 100 to 200°C, and preferably about 125 to 175°C, pressures of from about 500 to 5000 psi, and preferably about 1000 to 3000 psi, may satisfactorily be employed. 1 psi is equivalent to 0.06894757 bar.

The ratio of the weight of promoter and catalyst or catalyst mixture employed, to the weight of acetal, is desirably in the range of from about 0.001 to 0.1, and preferably 0.005 to 0.05 in a batch reaction.

In a further embodiment of this invention, it has been found that highly advantageous effects may also be obtained in this dealkoxyhydroxymethylation process by the addition of solvents in combination with the catalyst systems of this invention.

The solvent which may be advantageously used comprise any polar or non-polar organic solvents which are inert to the conditions of the reaction. Included amongst these solvents are $C_{1-12}$ alcohols, such as methanol, ethanol, butanol, and 3-ethyl-2--hexanol; ethers which will not cleave under the conditions of the reaction, such as glyme, diglyme, and diphenyl ether; aromatics and substituted aromatics such as benzene, toluene, xylene, chlorinated aromatic such as chlorobenzene and dichlorobenzene, and anisole.

The solvent may constitute anywhere from 0 to 90 volume percent of the reaction mixture, and preferably 20 to 80 percent.

In still a further embodiment of this process, it has been found that when the reaction is carried out in an excess of an alcohol solvent, wherein the ratio of acetal to alcohol solvent is in the range of from about 1:2 to 1:20, and preferably 1:5 to 1:10, and wherein the R group of the alcohol used is different than the $R^1$ and/or $R^2$ substituents on the acetal starting material, these different R groups of the alcohol will, in the course of the reaction, replace the $R^1$ and/or $R^2$ groups on the acetal in a substitution reaction, thereby resulting in a glycol monoether in which the R group of the ether moiety corresponds to the R group of the alcohol solvent.

This reaction may be illustrated by the following equation:

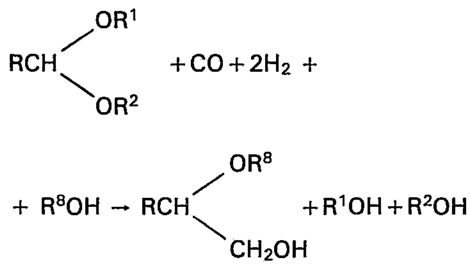


wherein R, $R^1$, and $R^2$ are as defined above, and $R^8$ is a different alkyl, cycloalkyl, or aryl group than $R^1$ and/or $R^2$, and having from 1 to about 20 carbon atoms. $R^8$ is preferably lower alkyl.

Depending upon the length of the time the reaction is allowed to continue, intermediate mixtures of higher and lower molecular weight substituents on the acetal corresponding to both those of the $R^1$ and/or $R^2$ groups and those of the solvent will be found in the reaction mixture.

The acetal starting materials employed in this invention have the aforedescribed general formula, namely

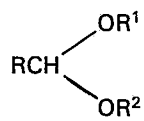

wherein the R, $R^1$ and $R^2$ are as defined above. These acetals can be prepared in a known manner as for example as described in E.V. Dehmlav and J. Schmidt, Tetrahedron Letters, p. 95-6 (1976) B.S. Bal and H.W. Pinnick, J. Org. Chem. V44 (21), p. 3727-8 (1979) D.W. Hall, U.S. Patent 3,492,356, Jan. 27 (1970), by the reaction of an aldehyde such as formaldehyde with an alcohol, or mixture of alcohols, of the general formula $R^1OH$ or $R^2OH$, where again $R^1$ and $R^2$ are as defined above, to form the corresponding acetal. Hereinafter, when the acetal is referred to, it will be understood that the corresponding precursors, i.e., the desired aldehyde and alcohol, are also intended to be included.

As also mentioned above, the $R^1$ and $R^2$ substituents of the resulting acetal may be joined by one or more bridging atoms to form such cyclic compounds as

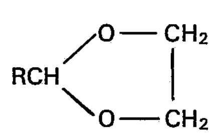

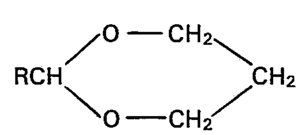

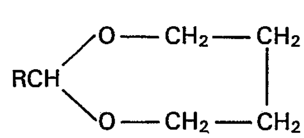

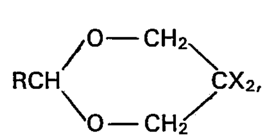,

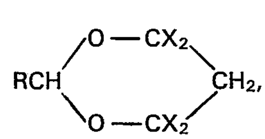,

and the like, wherein R is as defined above, and wherein X is selected from the group consisting of alkyl, aralkyl, aryl and cycloalkyl groups having from 1 to about 20 carbon atoms.

It is essential, in selecting the acetal starting material, that it not contain any substituents which are reactive under the conditions of the dealkoxyhydroxymethylation process of this invention. In other words, the R, $R^1$ and $R^2$ groups should not, for example, contain or comprise such reactive moieties as phosphine, arsine, amino, sulfido or carbonyl groups, acetal moieties, or olefins or acetylenic triple bonds. Other like groups will be recognized or readily determined by those skilled in the art as resulting in products other than the desired monoethers.

When, for example, formaldehyde acetals are dealkoxyhydroxymethylated with syngas in accordance with the process of this invention, there is obtained the corresponding ethylene glycol monoether in which the ether moiety will correspond to the organic moieties of the acetal starting material. Also formed in lesser amounts is the trialkoxyethane of the general formula

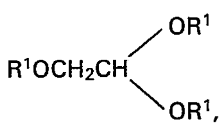

together with the alcohol $R^2OH$, which may be recycled to form additional acetal starting material. Again, as above, if the R groups of the acetal are different, a mixture of corresponding R-substituted compounds will result.

As shown below, the selectivities for the desired monoether over the trialkoxy by-product are in the ratio of from about 3:1 to as much as 10:1 or more.

In a preferred embodiment of this invention, the starting materials are preferably symmetrical acetals where the $R^1$ and $R^2$ groups are lower alkyl groups of 1 to 4 carbon atoms, thereby forming the corresponding ethylene glycol mono-lower alkyl ethers such as the monomethyl ether, the monoethyl ether, the monobutyl ether, and the like.

Alternatively, the acetal may contain such $R^1$ and $R^2$ groups as naphthyl and phenyl. In the case of naphthyl, the reaction of the resulting formaldehyde acetal with syngas will provide 2-(2-naphthyloxy) ethanol, a known sedative, which in turn may be oxidized to the corresponding 2-naphthyloxyacetic acid, a plant growth hormone.

Likewise, the dealkoxyhydroxymethylation of the formaldehyde acetal wherein $R^1$ and $R^2$ are phenyl will produce 2-phenoxy-ethanol, a topical antiseptic, which when oxidized, results in phenoxyacetic acid, a fungicide. Similarly, the formaldehyde acetal wherein $R^1$ and $R^2$ are 2, 4, 5-trichlorophenyl will yield, in accordance with this process, 2, 4, 5-trichlorophenoxyacetic acid, an herbicide. In a like manner, when $R^1$ and $R^2$ p-nonylphenyl, p-nonylphenoxyacetic acid, a corrosion inhibitor and antifoaming agent in gasoline and cutting oils will be formed.

Each of the aforedescribed products may be recovered routinely by methods well known in the art.

The invention will now be illustrated by, bus is not intended to be limited to, the following examples.

*Examples 1-6*

A series of runs was carried out wherein 285 mmoles of methylal were reacted with various catalyst combinations, as shown in Table I below, in a closed reactor at 150°C under 3000 psi of (2/l) syngas for 5 hours. As demonstrated by the results, the yield of ethylene glycol monomethyl ether (EGMME), was significantly enhanced by the presence of the phosphite promoter.

*Table I*

*METHYLAL DEALKOXYHYDROXYMETHYLATION[(a)]*

| CATALYST SYSTEM, MMOLES Example | $[HCCo_3(CO)_9]$ | $[Ru_3(CO)_{12}]$ | $[P(O\ neo\text{-}C_5H_{11})_3]^{(b)}$ | SELECT TO EGMME[(c)] % | CONV. OF METHYLAL % |
|---|---|---|---|---|---|
| 1 | 0 | 0 | 0 | 0 | 0 |
| 2 | 0.5 | 0 | 0 | 17 | 92 |
| 3 | 0.5 | 0.5 | 0 | 44 | 80 |
| 4 | 0.5[(d)] | 0.5 | 0 | 47 | 73 |
| 5 | 0.5 | 0.5 | 1.5 | 59 | 79 |
| 6 | 0.5[(d)] | 0.5 | 2.25 | 65 | 68 |

a) 285 mmoles of methylal reacted at 150°C under 3000 psi of 2/l syngas ($H_2/CO = 2/l$) for 5 hours.
b) Tri-neopentyl phosphite.
c) (Moles EGMME formed/moles methylal reacted) × 100.
d) Methylal dried over activated mole sieves before run.

*Examples 7-20*

A further series of runs was carried out wherein 4 mmoles of $Co_2(CO)_8$, 4 mmoles of $Ru_3(CO)_{12}$, trineopentyl phosphite and various solvents and reactants were charged together with butylal to a 300 ml autoclave, flushed 3 times with CO and $H_2$, brought to 3200 psig with a 1:1 mixture of CO and $H_2$ (syngas), heated to 150°C and stirred for 3 hours at that temperature. Analysis was based on a standardized GLPC of the liquid product. The results are shown in Table II below.

*Table II*

*Effects of Solvents and Added Phosphite on Dealkoxyhydroxymethylation of Butylal to Form Ethylene Glycol Monobutyl Ether (EGMBE)*

| Example | Solvent (Ml) | $CH_2(OBu)_2$ (Ml) | $(neo\text{-}C_5H_{11}O)_3P$ (MMole) | GC Analysis of Reaction Mixture, % | | | | | | | | Conv. of $CH_2(OBu)_2$ (Mole %) | Select. To EGMBE (Mole%) | Yield of EGMBE (Mole%) |
|---|---|---|---|---|---|---|---|---|---|---|---|---|---|---|
| | | | | $CH_3OBu$ | $PhCH_3$ [(b)] | BuOH | $CH_2(OBu)_2$ | EGMBE[(c)] | TBE[(d)] | Un-identified Heavies | Solvent | | | |
| 7 | None - 0 | 90 | 0 | 6.0 | 19.1 | 33.3 | 6.7 | 18.8 | 4.3 | 7.3 | | 94 | 32 | 30 |
| 8 | None - 0 | 90 | 3 | 6.9 | 17.6 | 38.8 | 6.4 | 23.1 | 2.7 | 4.4 | | 94 | 43 | 40 |
| 9 | n-BuOH - 45 | 45 | 0 | 2.5 | 17.2 | 61.6 | tr | 13.7 | 3.2 | 1.8 | | >99 | 44 | 44 |
| 10 | n-BuOH - 67 | 23 | 0 | 0.9 | 16.4 | 73.8 | tr | 4.8 | 1.0 | 3.2 | | >99 | 28 | 28 |
| 11 | N-BuOH - 45 | 45 | 3 | 1.9 | 14.5 | 63.0 | — | 17.0 | 1.0 | 1.5 | | >99 | 76 | 76 |
| 12 | n-BuOH - 45 | 45 | 6 | 2.0 | 14.5 | 61.8 | tr | 16.6 | 1.0 | 2 | | >99 | 72 | 72 |
| 13 | n-BuOH - 67 | 23 | 3 | 0.7 | 15.5 | 73 | | 8.4 | 0.3 | 1.2 | | >99 | 67 | 67 |
| 14 | $o\text{-}Cl_2C_6H_4$ - 67 | 23 | 0 | 0.9 | 18.0 | 5.7 | tr | 5.0 | 0.3 | 0.9 | 69.1 | 100 | 47 | 42 |
| 15 | $o\text{-}Cl_2C_6H_4$ - 45 | 45 | 3 | 1.4 | 15.5 | 16.0 | 1.0 | 13.2 | 1.1 | tr | 51.9 | >97 | 68 | 70 |
| 16 | $o\text{-}Cl_2C_6H_4$ - 67 | 23 | 3 | 0.8 | 16.0 | 6.7 | 0 | 7.3 | 0 | 0 | 69.1 | 100 | 76 | 76 |
| 17 | $o\text{-}Cl_2C_6H_4$ - 67 | 23 | 6 | 0.8 | 15.4 | 6.2 | 0 | 7.4 | tr | tr | 67.5 | >99 | 81 | 81 |
| 18 | $PhCH_3$ - 45 | 45 | 3 | 7.9 | (7.8) | 14.1 | 3.2 | 12.9 | tr | tr | 58.6 | 92 | 64 | 58 |
| 19 | $PhOCH_3$ - 67 | 23 | 6 | 0.6 | 13.2 | 6.4 | 0 | 6.7 | tr | tr | 72.4 | >99 | 70 | 70 |
| 20 | $PhOCH_3$ - 45 | 45 | 3 | 2.0 | 14.1 | 15.8 | 0 | 14.8 | tr | tr | 53.2 | >99 | 71 | 71 |

(a) methoxybutane (by-product)
(b) toluene (added internal GC standard)
(c) EGMBE = ethylene glycol monobutyl ether
(d) tri-butoxyethane (by-product).

*Examples 21-32*

In accordance with the general procedures of the foregoing examples, 70.6 mmoles of methylal, and 23.6 g. of o-dichlorobenzene, together with syngas (1:3 of $CO:H_2$) and various amounts of $HCCo_3(CO)_9$, $Ru_3(CO)_{12}$, tri-cyclohexylphosphite were charged to an autoclave at a maximum pressure of 3100-3200 psi for 5-6 hours at 150°C. The results are shown in Table III below.

*Table III*

*Phosphite-Promoted Dealkoxyhydroxymethylations of Methylal in the Presence of $HCCo_3(CO)_9$ and $Ru_3(CO)_{12}$*

| EXAMPLE | (a) | (b) | (c) | EGGME YIELD % | EGGME SELECT. % | METHYLAL CONV. % |
|---|---|---|---|---|---|---|
| 21 | .5 | 1.0 | 3.0 | 60 | 65 | 93 |
| 22 | .5 | .7 | 3.75 | 49 | 54 | 91 |
| 23 | .5 | .7 | 2.25 | 53 | 58 | 92 |
| 24 | .25 | .35 | 1.5 | 41 | 57 | 72 |
| 25 | .25 | .35 | 1.5 | 54 | 58 | 93 |
| 26 | .25 | .35 | 1.5 | 49 | 57 | 86 |
| 27 | .50 | 1.0 | 3.0 | 47 | 59 | 80 |
| 28 | .50 | .7 | 3.0 | 27 | 48 | 57 |
| 29 | .5 | .7 | 3.0 | 58 | 63 | 92 |
| 30 | .5 | 1.0 | 3.0 | 62 | 64 | 97 |
| 31 | .5 | 1.3 | 3.0 | 57 | 59 | 97 |
| 32 | 1.0 | 2.0 | 6.0 | 60 | 61 | 98 |

a) $HCCo_3(CO)_9$, mmoles    b) $Ru_3(CO)_{12}$, mmoles    c) tri-cyclohexylphosphite, mmoles.

*Example 33*

In accordance with the procedures of Example 17 but substituting $RuCo_2(CO)_{11}$ for the 1:1 mixture of $Co_2(CO)_8$ and $Ru_3(CO)_{12}$, there are obtained high yields and selectivities of ethylene glycol monobutyl ether.

*Example 34*

In accordance with the procedures of Example 6, but substituting $RuCo_2(CO)_{11}$ for the mixture of $HCCo_3(CO)_9$ and $Ru_3(CO)_{12}$, there are obtained high yields and selectivities of ethylene glycol monomethyl ether.

*Example 35*

In accordance with the procedures of Example 34, but substituting diethoxymethane for methylal one obtains high yields of ethylene glycol monoethyl ether.

*Example 36*

In accordance with the procedures of Example 21, except that triphenyl phosphite is substituted for trineopentylphosphite, high yields of ethylene glycol monomethyl ether are obtained.

*Examples 37-39*

A series of runs was carried out in which the following general procedure was employed using as the catalyst $R^3CCo_3(CO)_9$, as shown in Table IV below, in combination with $Ru_3(CO)_{12}$ and tri-neopentylphosphite. $R^3$ was varied from hydrogen, to phenyl ($C_6$-$H_5$-) to cyclooctenyl ($C_8H_{13}$-).

Reactions were carried out at 150°C in a 110 ml rocker bomb with 25 ml of liquid charge and 800 psi CO/1600 psi $H_2$ added initially. After the reaction, the mixture was analyzed by standardized gas chromatography.

*Table IV*

*Methylal Dealkoxyhydroxymethylation[a]*

| Example | Cobalt Complex | Ethylene Glycol monomethyl Ether (Yield, Mole %) |
|---|---|---|
| 37 | $C_6H_5CCo_3(CO)_9$ | 45 |
| 38 | $HCCo_3(CO)_9$ | 42 |
| 39 | $C_8H_{13}CCo_3(CO)_9$ | 39 |

a) $R^3CCo_3(CO)_9$, .5 mmoles; $Ru_3(CO)_{12}$, .5 mmoles; trineopentylphosphite, 0.75 mmoles; methylal, 25 ml; 800 psi CO; 1600 psi $H_2$; 150°C; 5 hrs.

*Examples 40-46*

To a 110 ml rocking autoclave was charged the catalyst system; acetal; o-dichlorobenzene (23.55 gms.); mesitylene internal standard (1.18 gms.); and CO and $H_2$. The reaction mixture was heated to the temperature shown below, and rocked for the designated time. The reaction mixture was cooled and the liquid product analyzed by standardized gas chromatography. The results are shown in Table V below.

*Table V*

*Dealkoxyhydroxymethylation of Formaldehyde Dialkyl Acetals*

| Example | Cobalt Catalyst-mmoles | Ruthenium Promoter-mmoles | Trialkyl Phosphite-mmoles | Acetal-mmoles | Reaction Temp., °C | Reaction Time, Min. | Init. CO. Pressure | Init. $H_2$+CO Pressure | EGMBE[1] % Yield | EGMBE[1] % Selec. | EGMBE[1] % Conv. |
|---|---|---|---|---|---|---|---|---|---|---|---|
| 40 | $HCCo_3(CO)_9$ - 0.5 | $Ru_3(CO)_{12}$ - 1.0 | Tricyclohexyl-3.0 | $CH_2(OC_4H_9)_2$-32.8 | 150 | 315 | 818 | 2417 | 85 | 85 | 100 |
| 41 | $HCCo_3(CO)_9$ - 0.5 | $Ru_3(CO)_{12}$ - 1.0 | Tricyclohexyl-3.0 | $CH_2(OC_4H_9)_2$-32.8 | 150 | 300 | 819 | 2379 | 86 | 87 | 99 |
| 42 | $HCCo_3(CO)_9$ - 0.5 | $Ru_3(CO)_{12}$ - 1.0 | Tricyclohexyl-3.0 | $CH_2(OC_4H_9)_2$-32.8 | 150 | 344 | 819 | 3207 | 92 | 97 | 95 |
| 43 | $HCCo_3(CO)_9$ - 0.5 | $Ru_3(CO)_{12}$ - 1.0 | Tricyclohexyl-3.0 | $CH_2(OC_4H_9)_2$-32.8 | 150 | 465 | 820 | 3242 | 90 | 95 | 96 |
| 44 | $HCCo_3(CO)_9$ - 0.5 | $Ru_3(CO)_{12}$ - 1.0 | Tricyclohexyl-3.0 | $CH_2(OC_4H_9)_2$-32.8 | 150 | 379 | 1215 | 2823 | 86 | 86 | 100 |
| 45 | $HCCo_3(CO)_9$ - 0.5 | $Ru_3(CO)_{12}$ - 1.0 | Tricyclohexyl-3.0 | $CH_2(OC_2H_5)_2$-50.6 | 150 | 300 | 820 | 2395 | 82 | 82 | 99 |
| 46 | $HCCo_3(CO)_9$ - 0.5 | $Ru_3(CO)_{12}$ - 1.0 | Tricyclohexyl-3.0 | $CH_2(OC_2H_5)_2$-50.6 | 125 | 215 | 821 | 2388 | 65 | 70 | 92 |

(1) EGMBE = ethylene glycol monobutyl ether.

*Example 47*

Using procedures similar to those employed in Example 42 but substituting propionaldehyde dibutylacetal for formaldehyde dibutylacetal, the monobutyl ether of butylene glycol is produced in good yield.

*Example 48*

Using the procedures of Example 47 but substituting acetaldehyde for propionaldehyde dibutylacetal and butanol for o-dichlorobenzene, the monobutyl ether of propylene glycol is produced in fair yield.

*Example 49*

Using the procedures of Example 48 but substituting paraformaldehyde for acetaldehyde, the monobutyl ether of ethylene glycol is produced in fair yield.

*Examples 50-54*

To a 110 ml rocking autoclave was charged the

catalyst system; acetal; o-dichlorobenzene (23.55 gms.); mesitylene internal standard (1.18 gms.); and CO and $H_2$. The reaction mixture was heated to the temperature shown below and rocked for the designated time. The reaction mixture was cooled and the liquid product analyzed by standard gas chromatography. The results are shown in Table VI below.

*Table VI*

*Dealkoxyhydroxymethylation of Acetaldehyde Diethyl Acetal*

| Example | Cobalt Catalyst-mmoles | Ruthenium Promoter-mmoles | Trialkyl Phosphite-mmoles | Acetal-mmoles | Reaction Temp., °C | Reaction Time, Min. | Init. CO. Pressure | Init. $H_2$+CO Pressure | PGMEE[b] % Yield | PGMEE[b] % Selec. | PGMEE[b] % Conv. |
|---|---|---|---|---|---|---|---|---|---|---|---|
| 50 | $HCCo_3(CO)_9$ - 0.5 | $Ru_3(CO)_{12}$ - 1.0 | Tricyclohexyl-3.0 | $CH_3CH(OC_2H_5)_2$-44.3 | 150 | 330 | 816 | 2402 | 35 | 35 | 99 |
| 51 | $HCCo_3(CO)_9$ - 0.5 | $Ru_3(CO)_{12}$ - 1.0 | Tricyclohexyl-3.0 | $CH_3CH(OC_2H_5)_2$-44.3 | 150 | 185 | 1616 | 3214 | 36 | 39 | 93 |
| 52 | $HCCo_3(CO)_9$ - 0.5 | $Ru_3(CO)_{12}$ - 1.0 | Tricyclohexyl-3.0 | $CH_3CH(OC_2H_5)_2$-44.3 | 150 | 1070 | 819 | 3203 | 37 | 47 | 100 |
| 53 | $HCCo_3(CO)_9$ - 0.5 | $Ru_3(CO)_{12}$ - 1.0 | Tricyclohexyl-3.0 | $CH_3CH(OC_2H_5)_2$-44.3 | 150 | 460 | 821 | 3209 | 47 | 49 | 100 |
| 54[a] | $HCCo_3(CO)_9$ - 0.5 | $Ru_3(CO)_{12}$ - 1.0 | Tricyclohexyl-3.0 | $CH_3CH(OC_2H_5)_2$-44.3 | 150 | 290 | 812 | 3207 | 50 | 50 | 100 |

(a) A 300 ml rocking autoclave was used for this run.
(b) PGMEE = propylene glycol monoethyl ether.

*Examples 55-57*

To a 110 ml rocking autoclave was charged dicobalt octacarbonyl (0.5 mmoles), triruthenium dodecacarbonyl (1.0 mmole), a phosphite (3.0 mmoles), the acetal (27 mmoles), the alcohol (18.2 ml), mesitylene as an internal standard, CO (800 psi), and $H_2$ to 3200 psi. The reaction mixture was heated to 150°C and rocked for 6 hours, after which it was cooled and the liquid product analyzed by standardized GC. The results are reported in Table VII below.

*Table VII*

*Dealkoxyhydroxymethylation of Acetals in Alcohol Solvents*

| Example | Phosphite | Acetal | Alcohol | Major Product | Yield % | Selec. % | Conv. % |
|---|---|---|---|---|---|---|---|
| 55 | $(cyclo\text{-}C_6H_{12}O)_3P$ | diethoxyethane | n-BuOH[a] | $CH_3CH(OBu)CH_2OH$ | b | b | b |
| 56 | $(cyclo\text{-}C_6H_{12}O)_3P$ | methylal | n-BuOH | $CH_2(OBu)CH_2OH$ | 59 | 59 | 99 |
| 57 | $(HO)_3P$ | methylal | n-BuOH | $CH_2(OBu)CH_2OH$ | 26 | 27 | 96 |

a - n-butyl alcohol
b - quantitative data unavailable.

*Example 58*

Following the procedures of Example 42 but substituting dibutylphosphite for tricyclohexyl phosphite, ethylene glycol monobutyl ether is obtained in excellent yield.

*Example 59*

Following the procedures of Example 42 but substituting diethylphosphite for tricyclohexylphosphite, EGMBE is prepared in high yield.

*Example 60*

Following the procedures of Example 54 but substituting dimethylphosphite for tricyclohexylphosphite there is obtained the monoethylether of propylene glycol in good yield.

*Examples 61-66*

A series of runs was carried out in which the following general procedure was employed, using as the catalyst $R^3CCo_3(CO)_9$ alone or promoted with $Ru_3(CO)_{12}$.

To a 300 ml stainless steel autoclave equipped with a magnedrive stirrer was charged: methylal, and catalyst. Carbon monoxide and hydrogen were admitted and the reaction mixture was rapidly heated to the desired temperature. The mixture was stirred for the designated time at reaction temperature after which the reactor was cooled by immersion in an ice bath. When the contents reached 25°C the final pressure was recorded. After venting the gas the liquid was analyzed by GLPC.

The results are reported in Table VIII below. The specific reaction conditions, amounts, and the use of solvents are described in footnote (a) in this table.

*Table VIII*

*METHYLAL DEALKOXYHYDROXYMETHYLATION*[(a)]

| EXAMPLES | CATALYST USED, MMOLES $[HCCo_3(CO)_9]$ | $[C_8H_{13}CCo_3(CO)_9]$ | $[PhCCo_3(CO)_9]$ | $[Ru_3(CO)_{12}]$ | YIELD EGMME[(b)] % | CONV. OF METHYLAL % |
|---|---|---|---|---|---|---|
| 61 | 0.5 | 0 | 0 | 0 | 17 | 92 |
| 62 | 0.5 | 0 | 0 | 0.5 | 44 | 80 |
| 63 | 0.5[(c)] | 0 | 0 | 0.5 | 47 | 73 |
| 64 | 0 | 0.5 | 0 | 0 | 20 | 63 |
| 65 | 0 | 0.5 | 0 | 0.5 | 42 | 79 |
| 66 | 0 | 0 | 0.5 | 0.5 | 35 | 63 |

(a) 285 mmoles of methylal reacted at 105°C under 3000 psi of 2/l syngas ($H_2/CO = 2/l$) for 5 hours
(b) (Moles EGMME formed/moles methylal reacted) × 100
(c) Methylal dried over activated mole sieves before run.

*Example 67*

In a manner similar to that described in Example 62 except that formaldehyde diethyl acetal is used in place of methylal, the monoethyl ether of ethylene glycol is formed in good yield.

*Example 68*

In a manner similar to that described in Example 62 except that formaldehyde dibutyl acetal is used in place of methylal, the monobutyl ether of ethylene glycol is formed in high yield.

*Example 69*

In a manner similar to that described in Example 62 except that acetaldehyde diethyl acetal is used in place of methylal, the monoethyl ether of propylene glycol is formed as a major reaction product.

*Example 70*

In a manner similar to that described in Example 69 except acetaldehyde, 285 mmoles, and ethanol, 470 mmoles, were used in place of acetaldehyde diethyl acetal, the monoethyl ether for propylene glycol was detected among the reaction products.

*Example 71*

To a 110 ml rocking autoclave is charged $HCCo(CO)_9$ (0.5 mmole), $Ru_3(CO)_{12}$ (1.0 mmole), methylal, (27 mmoles), butanol (18.2 mmoles), and mesitylene as internal standard. Carbon monoxide (800 psig) is charged to the reactor followed by hydrogen to a total pressure of 3200 psig and the mixture rocked at 150°C for 6 hours. Standardized gc of the reaction mixture after cooling shows that the monobutyl ether of ethylene glycol is formed in good yield.

**Claims**

1. A process for the dealkoxyhydroxymethylation or dearyloxyhydroxymethylation of an aldehyde acetal of the formula

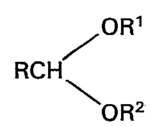

wherein R, $R^1$ and $R^2$, which may be same or different, are selected from alkyl, cycloalkyl, and aryl moieties having from 1 to 20 carbon atoms, wherein R may additionally be hydrogen and wherein $R^1$ and $R^2$, taken together, may be joined by one or more bridging atoms to form a cyclic acetal,
to form the corresponding glycol monoether, which comprises reacting said acetal, which has been formed separately or in situ with syngas in the presence of a catalytically effective amount of a cobalt-containing catalyst system, characterised in that the catalyst system comprises:

(a) $R^3CCo_3(CO)_9$ alone, or promoted with $Ru_3(CO)_{12}$; or

(b) an organophosphite of the formula

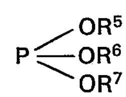

wherein $R^5$, $R^6$, and $R^7$, which may be the same or different, are selected from hydrogen and alkyl, cycloalkyl, and aryl moieties having from 1 to 20 carbon atoms; and
a ruthenium promoted cobalt carbonyl compound or mixture selected from:

1) a mixture of $Co_2(CO)_8$ and $Ru_3(CO)_{12}$;
2) $Co_2Ru(CO)_{11}$; and
3) a mixture of $R^3CCo_3(CO)_9$ promoted with $Ru_3(CO)_{12}$,

wherein $R^3$ is hydrogen; $C_{1-12}$ alkyl; $C_{5-10}$ cycloalkyl or alkyl-substituted cycloalkyl; $C_{6-12}$ cycloalkenyl; $C_{1-12}$ alkoxy; $C_{6-20}$ aryl or alkyl-, cycloalkyl-, alkoxy-, halo-, or cyano-substituted aryl; cyano; or a silyl carbyne moiety of the formula $R_3{}^4Si$, wherein $R^4$ is alkyl or aryl.

2. A process according to claim 1, wherein the mole ratio of organophosphite to ruthenium-cobalt carbonyl compound or mixture is in the range of from 1:5 to 5:1.

3. A process according to claim 1 or 2, wherein the phosphite is trineopentylphosphite.

4. A process according to any of claims 1 to 3, wherein the operating temperature is from 100 to 200°C.

5. A process according to any of claims 1 to 4, further comprising carrying out the reaction in the presence of an inert solvent.

6. A process according to claim 5, wherein the solvent is any polar or non-polar solvent which is inert to the conditions of the reaction.

7. A process according to claim 6, wherein the solvent is a $C_{1-12}$ alcohol; an ether, or an aromatic or substituted aromatic.

8. A process according to claim 5, wherein the solvent is a chlorinated aromatic solvent.

9. A process according to claim 8, wherein the solvent is chlorobenzene or dichlorobenzene.

10. A process according to any of claims 5 to 9, wherein the solvent constitutes up to 90 volume percent of the reaction mixture.

11. A process according to any of claims 1 to 10, wherein R, $R^1$ and $R^2$ are alkyl groups having from 1 to 20 carbon atoms.

12. A process according to claim 11, wherein the alkyl groups are lower alkyl.

13. A process according to any of claims 1 to 12, wherein the weight ratio of catalyst to acetal is in the range of from 0.001 to 0.1.

14. A process according to any of claims 1 to 13, wherein the organophosphite is trineopentylphosphite and the ruthenium-cobalt carbonyl compound is $Co_2Ru(CO)_{11}$.

15. A process according to any of claims 1, 2 and 4 to 13, wherein the organophosphite is cyclohexyl phosphite and the ruthenium-cobalt carbonyl compound is a mixture of $HCCo_3(CO)_9$ and $Ru_3(CO)_{12}$.

16. A process according to any of claims 1 to 15, wherein R is hydrogen, and the product is an ethylene glycol monoether; wherein R is methyl, and the product is a propylene glycol monoether; or wherein R is ethyl, and the product is a butylene glycol monoether.

17. A process according to any of claims 1, 2, 4 to 13 and 16, wherein the phosphite is tricyclohexylphosphite, tributylphosphite, triethylphosphite, trimethylphosphite or tri-2-ethylhexylphosphite.

18. A process according to any of claims 1 to 4 and 11 to 17, further comprising carrying out the reaction in the presence of an excess of an alcohol solvent of the formula

$$R^8OH$$

wherein $R^8$ is an alkyl, cycloalkyl, or aryl group having from 1 to 20 carbon atoms, and wherein $R^8$ is different from either the $R^1$ or $R^2$ group of the acetal starting material, or both, to form a glycol ether of the formula

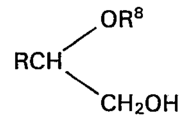

wherein $R^8$ is as defined above.

19. A process according to claim 18, wherein $R^8$ is lower alkyl.

20. A composition consisting essentially of a mixture of

(I) an organophosphite of the formula

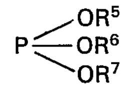

wherein $R^5$, $R^6$, and $R^7$, which may be the same or different, are selected from hydrogen and alkyl, cycloalkyl, and aryl moieties having from 1 to 20 carbon atoms; and

(II) a ruthenium promoted cobalt carbonyl compound or mixture selected from:

1) a mixture of $Co_2(CO)_8$ promoted with $Ru_3(CO)_{12}$;

2) $Co_2Ru(CO)_{11}$; and

3) a mixture of $R^3CCo_3(CO)_9$ and $Ru_3(CO)_{12}$, wherein $R^3$ is hydrogen; $C_{1-12}$ alkyl; $C_{5-10}$ cycloalkyl or alkyl-substituted cycloalkyl; $C_{6-12}$ cycloalkenyl; $C_{1-12}$ alkoxy; $C_{6-20}$ aryl or alkyl-, cycloalkyl-, alkoxy-, halo-, or cyano-substituted aryl; cyano; or a silyl carbyne moiety of the formula $R_3{}^4Si$, wherein $R^4$ is alkyl or aryl.

21. A composition according to claim 20, wherein the phosphite is a trialkylphosphite, tricycloalkylphosphite, triarylphosphite, dialkylphosphite, dicycloalkylphosphite or triarylphosphite.

22. A composition according to claim 20 or 21, consisting essentially of:

$Co_2(CO)_8$, $Ru_3(CO)_{12}$, and $(neo\text{-}C_5H_{11}O)_3P$;
$Co_2(CO)_8$, $Ru_3(CO)_{12}$, and $(cyclo\text{-}C_6H_{12}O)_3P$;
$Co_2(CO)_8$, $Ru_3(CO)_{12}$, and $(n\text{-}C_4H_9O)_3P$;
$HCCo_3(CO)_9$, $Ru_3(CO)_{12}$, and $(neo\text{-}C_5H_{11}O)_3P$;
$HCCo_3(CO)_9$, $Ru_3(CO)_{12}$, and $(cyclo\text{-}C_6H_{12}O)_3P$;
$HCCo_3(CO)_9$, $Ru_3(CO)_{12}$, and $(n\text{-}C_4H_9O)_3P$;
$Co_2(CO)_8$, $Ru_3(CO)_{12}$, and dimethylphosphite;
$Co_2(CO)_8$, $Ru_3(CO)_{12}$, and diethylphosphite;
$PhCCo_3(CO)_9$, $Ru_3(CO)_{12}$, and $(neo\text{-}C_5H_{11}O_3)_3P$, wherein Ph is phenyl;
$CH_3CCo_3(CO)_9$, $Ru_3(CO)_{12}$, and $(cyclo\text{-}C_6H_{12}O)_3P$; or
$cyclo\text{-}C_8H_{13}CCo_3(CO)_9$, $Ru_3(CO)_{12}$, and $(n\text{-}C_4H_9O)_3P$.

23. A composition comprising a mixture of $R^3CCo_3(CO)_9$ and $Ru_3(CO)_{12}$, wherein $R^3$ is hydrogen; $C_{1-12}$ alkyl; $C_{5-10}$ cycloalkyl or alkyl-substituted cycloalkyl; $C_{6-12}$ cycloalkenyl; $C_{1-12}$ alkoxy; $C_{6-20}$ aryl or alkyl-, cycloalkyl-, alkoxy-, halo-, or cyano-substituted aryl; cyano; or a silyl carbyne moiety of the formula $R_3{}^4Si$, wherein $R^4$ is alkyl or aryl.

24. A composition according to claim 23 wherein $R^3$ is hydrogen, cyclooctenyl or phenyl.

**Patentansprüche**

1. Verfahren zur Entalkoxyhydroxymethylierung oder Entaryloxyhydroxymethylierung eines Aldehydacetals der Formel

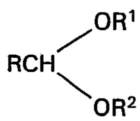

worin R, $R^1$ und $R^2$, die gleich oder verschieden sein können, unter Alkyl-, Cycloalkyl- und Arylgruppen mit 1 bis 20 Kohlenstoffatomen ausgewählt sind, wobei R zusätzlich Wasserstoff sein kann, und worin $R^1$ und $R^2$ miteinander über ein oder mehrere Brückenatome unter Bildung eines cyclischen Acetals verbunden sein können,
wobei der entsprechende Glycolmonoether gebildet wird, bei dem das Acetal, welches gesondert oder in situ hergestellt wurde, mit Synthesegas in Gegenwart einer katalytisch wirksamen Menge eines Cobalt enthaltenden Katalysatorsystems umgesetzt wird, dadurch gekennzeichnet, dass das Katalysatorsystem umfasst:

(a) $R^3CCo_3(CO)_9$ für sich oder mit $Ru_3(CO)_{12}$ als Promotor versehen; oder

(b) ein Organophosphit der Formel

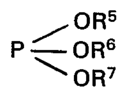

worin $R^5$, $R^6$ und $R^7$, die gleich oder verschieden sein können, unter Wasserstoff und Alkyl-, Cycloalkyl- und Arylgruppen mit 1 bis 20 Kohlenstoffatomen ausgewählt sind; und
eine mit Ruthenium als Promotor versehene Cobaltcarbonylverbindung oder ein Gemisch, das aus folgenden Gruppen ausgewählt ist:

1) einem Gemisch von $Co_2(CO)_8$ und $Ru_3(CO)_{12}$;
2) $Co_2Ru(CO)_{11}$; und
3) einem Gemisch von $R^3CCo_3(CO)_9$, mit $Ru_3(CO)_{12}$, als Promotor versehen,

worin $R^3$ für Wasserstoff; $C_{1-12}$-Alkyl; $C_{5-10}$-Cycloalkyl oder alkylsubstituiertes Cycloalkyl; $C_{6-12}$--Cycloalkenyl; $C_{1-12}$-Alkoxy; $C_{6-20}$-Aryl- oder -alkyl-, -cycloalkyl-, -alkoxy-, -halogen-, oder -cyan-substituiertes Aryl; eine Cyangruppe oder eine Silylcarbin-Gruppe der Formel $R_3^4Si$ steht, worin $R^4$ Alkyl oder Aryl bedeutet.

2. Verfahren nach Anspruch 1, bei dem das Molverhältnis von Organophosphit zu der Ruthenium-Cobaltcarbonylverbindung oder dem Gemisch im Bereich von 1:5 bis 5:1 liegt.

3. Verfahren nach Anspruch 1 oder 2, bei dem das Phosphit Trineopentylphosphit ist.

4. Verfahren nach einem der Ansprüche 1 bis 3, bei dem die Arbeitstemperatur 100 bis 200°C beträgt.

5. Verfahren nach einem der Ansprüche 1 bis 4, bei dem die Reaktion in Gegenwart eines inerten Lösungsmittels durchgeführt wird.

6. Verfahren nach Anspruch 5, bei dem das Lösungsmittel ein polares oder nicht polares Lösungsmittel ist, welches inert gegenüber den Bedingungen der Reaktion ist.

7. Verfahren nach Anspruch 6, bei dem das Lösungsmittel ein $C_{1-12}$-Alkohol, ein Ether oder eine aromatische oder substituierte aromatische Verbindung ist.

8. Verfahren nach Anspruch 5, bei dem das Lösungsmittel ein chloriertes aromatisches Lösungsmittel ist.

9. Verfahren nach Anspruch 8, bei dem das Lösungsmittel Chlorbenzol oder Dichlorbenzol ist.

10. Verfahren nach einem der Ansprüche 5 bis 9, bei dem das Lösungsmittel einen Anteil bis zu 90 Vol.-% des Reaktionsgemisches darstellt.

11. Verfahren nach einem der Ansprüche 1 bis 10, bei dem R, $R^1$ und $R^2$ Alkylgruppen mit 1 bis 20 Kohlenstoffatomen sind.

12. Verfahren nach Anspruch 11, bei dem die Alkylgruppen niedere Alkylgruppen sind.

13. Verfahren nach einem der Ansprüche 1 bis 12, bei dem das Gewichtsverhältnis von Katalysator zu Acetal im Bereich von 0,001 bis 0,1 liegt.

14. Verfahren nach einem der Ansprüche 1 bis 13, bei dem das Organophosphit Trineopentylphosphit und die Ruthenium-Cobaltcarbonyl-Verbindung $Co_2Ru(CO)_{11}$ ist.

15. Verfahren nach einem der Ansprüche 1, 2 und 4 bis 13, bei dem das Organophosphit Cyclohexylphosphit ist und die Ruthenium-Cobaltcarbonyl-Verbindung ein Gemisch aus $HCCo_3(CO)_9$ und $Ru_3(CO)_{12}$ ist.

16. Verfahren nach einem der Ansprüche 1 bis 15, bei dem R Wasserstoff ist und als Produkt ein Ethylenglycol-monoether erhalten wird; bei dem R Methyl ist und als Produkt ein Propylenglycol-monoether erhalten wird oder bei dem R Ethyl ist und als Produkt Butylenglycol-monoether erhalten wird.

17. Verfahren nach einem der Ansprüche 1, 2, 4 bis 13 und 16, bei dem das Phosphit Tricyclohexylphosphit, Tributylphosphit, Triethylphosphit, Trimethylphosphit oder Tri-2-ethylhexylphosphit ist.

18. Verfahren nach einem der Ansprüche 1 bis 4 und 11 bis 17, bei dem die Reaktion in Gegenwart eines Überschusses eines Alkohol-Lösungsmittels der Formel

$$R^8OH$$

durchgeführt wird, worin $R^8$ eine Alkyl-, Cycloalkyl- oder Arylgruppe mit 1 bis 20 Kohlenstoffatomen bedeutet und worin $R^8$ verschieden von entweder der Gruppe $R^1$ oder der Gruppe $R^2$ oder von beiden Gruppen des Acetal-Ausgangsmaterials ist, wobei ein Glycolether der Formel

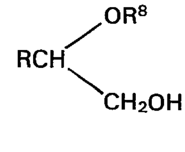

gebildet wird, in dem $R^8$ die vorstehende Definition hat.

19. Verfahren nach Anspruch 18, worin $R^8$ eine niedere Alkylgruppe ist.

20. Zusammensetzung, bestehend im wesentlichen aus einem Gemisch aus

(I) einem Organophosphit der Formel

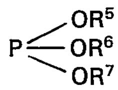

worin $R^5$, $R^6$ und $R^7$ gleich oder verschieden sein können und unter Wasserstoff und Alkyl-, Cycloalkyl- und Arylgruppen mit 1 bis 20 Kohlenstoffatomen ausgewählt sind; und

(II) eine mit Ruthenium als Promotor versehene Cobaltcarbonylverbindung oder ein Gemisch ausgewählt unter

1) einem Gemisch von $Co_2(CO)_8$ und $Ru_3(CO)_{12}$ als Promotor;

2) $Co_2Ru(CO)_{11}$; und

3) einem Gemisch von $R^3CCo_3(CO)_9$ und $Ru_3(CO)_{12}$,

worin $R^3$ für Wasserstoff; $C_{1-12}$-Alkyl; $C_{5-10}$-Cycloalkyl oder alkylsubstituiertes Cycloalkyl; $C_{6-12}$-Cycloalkenyl; $C_{1-12}$-Alkoxy; $C_{6-20}$-aryl- oder -alkyl-, -cycloalkyl-, -alkoxy-, -halogen-, oder -cyan-substituiertes Aryl; eine Cyangruppe oder eine Silylcarbin-Gruppe der Formel $R_3{}^4Si$ steht, worin $R^4$ Alkyl oder Aryl bedeutet.

21. Zusammensetzung nach Anspruch 20, worin das Phosphit ein Trialkylphosphit, Tricycloalkylphosphit, Triarylphosphit, Dialkylphosphit, Dicycloalkylphosphit oder Diarylphosphit ist.

22. Zusammensetzung nach Anspruch 20 oder 21, bestehend im wesentlichen aus:

$Co_2(CO)_8$, $Ru_3(CO)_{12}$ und $(Neo\text{-}C_5H_{11}O)_3P$;
$Co_2(CO)_8$, $Ru_3(CO)_{12}$ und $(Cyclo\text{-}C_6H_{12}O)_3P$;
$Co_2(CO)_8$, $Ru_3(CO)_{12}$ und $(n\text{-}C_4H_9O)_3P$;
$HCCo_3(CO)_9$, $Ru_3(CO)_{12}$ und $(Neo\text{-}C_5H_{11}O)_3P$;
$HCCo_3(CO)_9$, $Ru_3(CO)_{12}$ und $(Cyclo\text{-}C_6H_{12}O)_3P$;
$HCCo_3(CO)_9$, $Ru_3(CO)_{12}$ und $(n\text{-}C_4H_9O)_3P$;
$Co_2(CO)_8$, $Ru_3(CO)_{12}$ und Dimethylphosphit;
$Co_2(CO)_8$, $Ru_3(CO)_{12}$ und Diethylphosphit;
$PhCCo_3(CO)_9$, $Ru_3(CO)_{12}$ und $(Neo\text{-}C_5H_{11}O_3)_3P$, worin Ph Phenyl ist;
$CH_3CCo_3(CO)_9$, $Ru_3(CO)_{12}$, und $(Cyclo\text{-}C_6H_{12}O)_3P$; oder
$Cyclo\text{-}C_8H_{13}CCo_3(CO)_9$, $Ru_3(CO)_{12}$, und $(n\text{-}C_4H_9O)_3P$.

23. Zusammensetzung, umfassend ein Gemisch aus $R^3CCo_3(CO)_9$ und $Ru_3(CO)_{12}$, worin $R^3$ für Wasserstoff; $C_{1-12}$-Alkyl; $C_{5-10}$-Cycloalkyl oder -alkylsubstituiertes Cycloalkyl; $C_{6-12}$-Cycloalkenyl; $C_{1-12}$-Alkoxy; $C_{6-20}$-Aryl oder -alkyl-, -cycloalkyl-, -alkoxy-, -halogen- oder -cyansubstituiertes Aryl; Cyan oder eine Silylcarbin-Gruppe der Formel $R_3{}^4Si$ steht, wobei $R^4$ Alkyl oder Aryl bedeutet.

24. Zusammensetzung nach Anspruch 23, worin $R^3$ für Wasserstoff, Cyclooctenyl oder Phenyl steht.

**Revendications**

1. Procédé pour la déalkoxyhydroxyméthylation, ou la déaryloxyhydroxyméthylation, d'un acétal d'aldéhyde de formule:

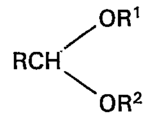

dans laquelle R, $R^1$ et $R^2$, qui peuvent être semblables ou différents, sont des groupements alkyle, cycloalkyle ou aryle ayant de 1 à 20 atomes de carbone, R pouvant être aussi de l'hydrogène, et dans laquelle $R^1$ et $R^2$, pris ensemble, peuvent être réunis par un ou plusieurs atomes formant un pont pour donner un acétal cyclique, en vue de la formation d'un monoéther de glycol correspondant, qui consiste à faire réagir cet acétal, qui a été formé séparément ou in situ, avec du syngas

en présence d'une quantité efficace d'un système catalytique renfermant du cobalt, caractérisé en ce que ce système catalytique comprend:

a) $R^3CCo_3(CO)_9$ seul ou activé par $Ru_3(CO)_{12}$; ou

b) un organophosphite de formule

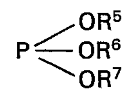

dans laquelle $R^5$, $R^6$ et $R^7$, qui peuvent être semblables ou différents, sont de l'hydrogène ou des groupements alkyle, cycloalkyle ou aryle, ayant de 1 à 20 atomes de carbone; et un composé de cobalt carbonyle activé au ruthénium, ou un mélange choisi à partir de:

1) un mélange de $Co_2(CO)_8$ et $Ru_3(CO)_{12}$;

2) $Co_2Ru(CO)_{11}$; et

3) un mélange de $R^3CCo_3(CO)_9$ activé par $Ru_3(CO)_{12}$,

dans lequel $R^3$ est hydrogène, alkyle en $C_1$ à $C_{12}$, cycloalkyle ou cycloalkyle substitué en $C_5$ à $C_{10}$, cycloalkényle en $C_6$ à $C_{12}$, alkoxy en $C_1$ à $C_{12}$, aryle ou aryle substitué par alkyle, cycloalkyle, alkoxy, halogène ou cyano, en $C_6$ à $C_{20}$, cyano, ou un groupement silyl carbyne de formule $R_3{}^4Si$; et dans lequel $R^4$ est alkyle ou aryle.

2. Procédé selon la revendication 1, dans lequel le rapport molaire de l'organophosphite au composé de ruthénium-cobalt carbonyle ou son mélange, est compris dans l'intervalle de 1/5 à 5/1.

3. Procédé selon une des revendications 1 ou 2, dans lequel le phosphite est le phosphite de trinéopentyle.

4. Procédé selon une des revendications 1 à 3, dans lequel on opère à une température allant de 100° à 200°C.

5. Procédé selon une des revendications 1 à 4, au cours duquel la réaction est en outre réalisée en présence d'un solvant inerte.

6. Procédé selon la revendication 5, dans lequel le solvant est un solvant polaire ou non polaire qui s'avère inerte dans les conditions de la réaction.

7. Procédé selon la revendication 6, dans lequel le solvant est un alcool en $C_1$ à $C_{12}$, un éther ou un composé aromatique ou aromatique substitué.

8. Procédé selon la revendication 5, dans lequel le solvant est un solvant aromatique chloré.

9. Procédé selon la revendication 8, dans lequel le solvant est le chloro- ou le dichloro-benzène.

10. Procédé selon une des revendications 5 à 9, dans lequel le solvant représente jusqu'à 90% en volume du mélange réactionnel.

11. Procédé selon une des revendications 1 à 10, dans lequel R, $R^1$ et $R^2$ sont des groupements alkyle ayant de 1 à 20 atomes de carbone.

12. Procédé selon la revendication 11, dans lequel les groupements alkyle sont des alkyle inférieurs.

13. Procédé selon une des revendications 1 à 12, dans lequel le rapport pondéral du catalyseur à l'acétal se situe dans l'intervalle de 0,001 à 0,1.

14. Procédé selon une des revendications 1 à 13, dans lequel l'organophosphite est le phosphite de trinéopentyle, et le composé de ruthénium-cobalt carbonyle est $Co_2Ru(CO)_{11}$.

15. Procédé selon une des revendications 1, 2 et 4 à 13, dans lequel l'organophosphite est le phosphite de cyclohexyle, et le composé de ruthénium-cobalt carbonyle est un mélange de $HCCo_3(CO)_9$ et de $Ru_3(CO)_{12}$.

16. Procédé selon une des revendications 1 à 15, dans lequel R est hydrogène, et le produit est un monoéther d'éthylène glycol; R est méthyle, et le produit est un monoéther de propylène glycol; ou R est éthyle, et le produit est un monoéther de butylène glycol.

17. Procédé selon une des revendications 1, 2, 4 à 13, et 16, dans lequel le phosphite est le phosphite de tricyclohexyle, de tributyle, de triéthyle, de triméthyle ou de tri-(éthyl-2 hexyle).

18. Procédé selon une des revendications 1 à 4 et 11 à 17, au cours duquel la réaction se déroule en outre en présence d'un excès d'un alcool solvant de formule

$$R^8OH$$

dans laquelle $R^8$ est un groupe alkyle, cycloalkyle ou aryle, ayant 1 à 20 atomes de carbone, et est différent des groupes $R^1$ ou $R^2$ ou des deux, de l'acétal de départ, avec formation d'un éther de glycol de formule

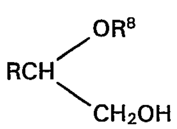

dans laquelle $R^8$ est comme défini plus haut.

19. Procédé selon la revendication 18, dans lequel $R^8$ est un alkyle inférieur.

20. Composition comprenant essentiellement un mélange de:

I) un organophosphite de formule

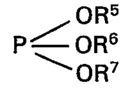

dans laquelle $R^5$, $R^6$ et $R^7$, qui peuvent être semblables ou différents, sont hydrogène ou radical alkyle, cycloalkyle ou aryle ayant de 1 à 20 atomes de carbone; et

II) un composé de cobalt carbonyle activé par du ruthénium ou un mélange choisi à partir de:

1) un mélange de $Co_2(CO)_8$ activé par $Ru_3(CO)_{12}$;

2) $Co_2Ru(CO)_{11}$; et

3) un mélange de $R^3CCo_3(CO)_9$ et $Ru_3(CO)_{12}$, dans lequel $R^3$ est hydrogène, alkyle en $C_1$ à $C_{12}$, cycloalkyle ou cycloalkyle substitué en $C_5$ à $C_{10}$, cycloalkényle en $C_6$ à $C_{12}$, alkoxy en $C_1$ à $C_{12}$, aryle ou aryle substitué par alkyle, cycloalkyle, alkoxy, ou groupement halogène ou cyano, en $C_6$ à $C_{20}$, groupement cyano, ou un radical silyl carbyne de formule $R_3{}^4Si$, dans laquelle $R^4$ est alkyle ou aryle.

21. Composition selon la revendication 20, dans laquelle le phosphite est un phosphite de trialkyle, de tricycloalkyle, de triaryle, de dialkyle, de dicycloalkyle ou de triaryle.

22. Composition selon la revendication 20 ou 21, qui comprend essentiellement:
$Co_2(CO)_8$, $Ru_3(CO)_{12}$ et $(néo\text{-}C_5H_{11}O)_3P$;
$Co_2(CO)_8$, $Ru_3(CO)_{12}$ et $(cyclo\text{-}C_6H_{12}O)_3P$;
$Co_2(CO)_8$, $Ru_3(CO)_{12}$ et $(n\text{-}C_4H_9O)_3P$;
$HCCo_3(CO)_9$, $Ru_3(CO)_{12}$ et $(néo\text{-}C_5H_{11}O)_3P$;
$HCCo_3(CO)_9$, $Ru_3(CO)_{12}$ et $(cyclo\text{-}C_6H_{12}O)_3P$;
$HCCo_3(CO)_9$, $Ru_3(CO)_{12}$ et $(n\text{-}C_4H_9O)_3P$;
$Co_2(CO)_8$, $Ru_3(CO)_{12}$, et phosphite de diméthyle;
$Co_2(CO)_8$, $Ru_3(CO)_{12}$, et phosphite de diéthyle;
$PhCCo_3(CO)_9$, $Ru_3(CO)_{12}$, et $(néo\text{-}C_5H_{11}O)_3P$,
dans laquelle Ph est phényle;
$CH_3CCo_3(CO)_9$, $Ru_3(CO)_{12}$ et $(cyclo\text{-}C_6H_{12}O)_3P$; ou
$cyclo\text{-}C_8H_{13}CCo_3(CO)_9$, $Ru_3(CO)_{12}$ et $(n\text{-}C_4H_9O)_3P$.

23. Composition qui comprend un mélange de $R^3CCo_3(CO)_9$ et de $Ru_3(CO)_{12}$, dans laquelle $R^3$ est hydrogène, alkyle en $C_1$ à $C_{12}$, cycloalkyle ou cycloalkyle substitué par un alkyle, en $C_5$ à $C_{10}$, cycloalkényle en $C_6$ à $C_{12}$, alkoxy en $C_1$ à $C_{12}$, aryle ou aryle substitué par alkyle, cycloalkyle, alkoxy, radical halogène ou cyano, en $C_6$ à $C_{20}$, radical cyano, ou radical silyl carbyne de formule $R_3{}^4Si$, dans laquelle $R^4$ est alkyle ou aryle.

24. Composition selon la revendication 23, dans laquelle $R^3$ est hydrogène, cyclooctényle ou phényle.